(19) Europäisches Patentamt
European Patent Office
Office européen des brevets

(11) Publication number: **0 152 031**
**B1**

(12) **EUROPEAN PATENT SPECIFICATION**

(45) Date of publication of patent specification: **09.05.90**

(21) Application number: **85101066.0**

(22) Date of filing: **01.02.85**

(51) Int. Cl.$^5$: **C 07 D 249/08,**
C 07 D 233/60,
C 07 D 409/08,
A 01 N 43/653, A 01 N 43/50

(54) **Azolyl cycloalkanol derivatives and agricultural fungicides.**

(30) Priority: **03.02.84 JP 18564/84**
**15.02.84 JP 27905/84**

(43) Date of publication of application:
**21.08.85 Bulletin 85/34**

(45) Publication of the grant of the patent:
**09.05.90 Bulletin 90/19**

(84) Designated Contracting States:
**CH DE FR IT LI NL SE**

(56) References cited:
**EP-A-0 000 951**
**EP-A-0 094 146**
**FR-A-2 198 752**

(73) Proprietor: **SHIONOGI & CO., LTD.**
**1-8, Doshomachi 3-chome Chuo-ku**
**Osaka 541 (JP)**

(72) Inventor: **Makisumi, Yasuo**
**2-5-17, Midoridai**
**Kawanishi-shi Hyogo (JP)**
Inventor: **Murabayashi, Akira**
**2-12-3, Kamichujo**
**Ibaraki-shi Osaka (JP)**
Inventor: **Hatta, Takayuki**
**116-38, Ohaza-sagami**
**Koga-cho Koga-gun Shiga (JP)**
Inventor: **Ishiguro, Takeo**
**2-16-43, Kusatsu**
**Kusatsu-shi Shiga (JP)**

(74) Representative: **Bruin, Cornelis Willem et al**
**Octrooibureau Arnold & Siedsma Isartorplatz 5**
**D-8000 München 2 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 152 031 B1

Courier Press, Leamington Spa, England.

**Description**

This invention relates to azolylcycloalkanol derivatives and their salts which have a strong antimicrobial and especially antifungal activity. Further, it relates to a method of preparing such derivatives and to the use thereof in preparing agricultural fungicides.

The derivatives of the present invention are cycloheptanol derivatives having an optionally halogen-substituted phenyl, benzyl, or thienyl group in 1-position and an imidazolyl or 1,2,4-triazolyl group (commonly referred to as an azolyl group) in 2-position. Moreover, the azolyl and hydroxyl groups attached to the cycloheptane nucleus are in cis-configuration.

The derivatives of the present invention are represented by the general formula (I)

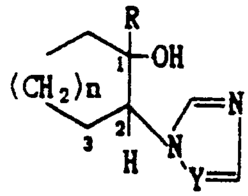

I

wherein R is a phenyl, benzyl or thienyl group optionally substituted by one or more halogen atoms, Y is a methine or nitrilo group and n is 3, with the proviso that the azolyl and hydroxyl groups attached to the cycloheptane ring are in cis-configuration. Salts with organic or inorganic acids are also included in the definition.

The derivatives of formula I and their salts have strong antimicrobial activity, especially against fungi infecting agricultural products. Therefore, they can be used for producing agricultural fungicides.

The state of the art comprised already some related azolylcycloalkanol derivatives of similar activity. Thus, antifungal 1-phenyl (or substituted phenyl)-2-azolyl-cycloalkanol derivatives have been disclosed in EP—A—0094146. The definition given in this document is wide enough to cover cycloheptanol as well as cyclohexanol and cyclopentanol derivatives but the only cycloalkanol derivatives exemplified therein are cyclohexanol derivatives. Moreover, the synthetic route disclosed in EP—A—0094146 must necessarily lead to derivatives having the azolyl and hydroxyl groups attached to the cycloalkane ring in trans-configuration and this is confirmed by the physical constants of the exemplified cyclohexanol derivatives.

In the derivatives of formula I, the halogen atoms may be located at any position of the phenyl or thienyl ring and the number of halogen atoms is not limited. Preferably, R is 2-chlorophenyl, 2-fluorophenyl, 3-chlorophenyl, 4-chlorophenyl, 4-fluorophenyl, 2,4-diclorophenyul, 2,5-dichlorophenyl, 3,4-dichlorophenyl, benzyl, 2-chlorobenzyl, 4-chlorobenzyl, 2,4-dichlorobenzyl, 4-fluorobenzyl, 2,4-difluorobenzyl, 2-thienyl, 5-chloro-2-thienyl, 5-iodo-2-thienyl, 3,5-dichloro-2-thienyl or 3,4,5-trichloro-2-thienyl. More preferable groups for R are 2-chlorophenyl, 4-chlorophenyl, 4-fluorophenyl, 2,5-dichlorophenyl, 5-chloro-2-thienyl and 3,4,5-trichloro-2-thienyl. Y may be methine and nitrilo and the nitrilo group is preferred.

The antifungal activity of the invented derivatives is illustrated by the following tests wherein the test compounds are represented by the same number as used in the working examples presented later on.

Test 1 Damping-off of cucumber seedlings

(1) The fungus causing damping-off of cucumber seedlings, *Rhizoctonia solani* or *Fusarium oxysporum* was cultured on a wheat bran medium of 28°C for 5 days. Thereupon, the medium was mixed with sterilized soil and cultured for 2 days to prepare the inoculum. Pots of 9 cm in diameter were filled with sterilized soil (150 g) and the surface of the soil was covered with the above inoculum. A solution (30 ml) of the test compound at a concentration of 500 ppm or 31,3 ppm was poured into each pot whereupon the pots were dried. Twenty cucumber seeds (Cultivar: Matsukaze) were sown 2 days later. The pots were kept at 30 ± 2°C for 14 days. The infection was observed 14 days later and percentages of disease degree and disease control were obtained by allotting evaluation indexes to each disease symptom.

(2) Evaluation

i. Disease degree for *Rhizoctonia solani*

$$= \frac{4a + 3b + 2c + d}{4(a + b + c + d + e)} \times 100$$

wherein

a: number of seeds without germination

b: number of seedlings showing damping-off

c: number of wilted seedlings

d: number of seedlings being a little brownish in their cotyledons or at the bottom of their stems
e: number of uninfected seedlings.

ii. Disease degree for *Fusarium oxysporum*

$$= \frac{3a + 2b + c}{3(a + b + c + d)} \times 100$$

wherein
a: number of seeds without germination or seedlings with damping-off
b: number of wilted seedlings
c: number of seedlings being a little brownish in their cotyledons or at the bottom of their stems
d: number of uninfected seedlings.

iii. Disease control (%)

$$= \frac{\text{Disease degree in untreated plot} - \text{Disease degree in treated plot}}{\text{Disease degree in untreated plot}} \times 100$$

(3) Results

TABLE 1

| Test Comp. | Concentration (ppm) | *Rhizoctonia solani* Disease Degree | *Rhizoctonia solani* Disease Control (%) | *Fusarium oxysporum* Disease Degree | *Fusarium oxysporum* Disease Control (%) |
|---|---|---|---|---|---|
| 3 | 500 | 5 | 95 | 100 | 0 |
| 7 | 31.3 | 18 | 82 | — | — |
| Captan | 800 | — | — | 0 | 100 |
| Mepronil | 750 | 5 | 95 | — | — |
| " | 47 | 77 | 23 | — | — |
| Untreated | — | 100 | 0 | 100 | 0 |

Test 2 Test on Botrytis rot (gray mold) of cucumber.

(1) Seedlings of cucumber (Cultivar: Matsukaze) were planted in pots of 9 cm in diameter. The first leaf was sprayed with 3 ml of a solution of the test compound at a concentration of 125 or 31.3 ppm and the plant was dried in the air. After inoculation with spores of *Botrytis cinerea,* the plants were kept at 20 ± 2°C and 90—100% RH ((i) protective spraying). Alternatively, the plants were inoculated first with Botrytis spores and the solutions of the test compounds were sprayed about 2 days later when the leaves had suffered a little ((ii) curative spraying). An evaluation was made 3 days after the inoculation. The percentage of disease control was calculated from the diameters of disease areas in treated and untreated plants.

(2) Evaluation
Disease control (%)

$$= \frac{\text{Diameter of diseased area in untreated plot} \qquad \text{Diameter of diseased area in treated plot}}{\text{Diameter of disease area in untreated plot}} \times 100$$

(3) Results

Table 2

| Test Compound | Concentration (ppm) | Disease Control (%) | |
|---|---|---|---|
| | | Protective Spraying | Curative Spraying |
| 3 | 125 | 100 | 94 |
| 5 | 125<br>31.3 | 100<br>100 | 94<br>86 |
| 7 | 125<br>31.3 | 100<br>89 | 94<br>86 |
| Iprodione | 125<br>31.3 | 100<br>100 | 90<br>64 |
| Untreated | - | 0 | 0 |

Test 3 Test on Sclerotinia rot of cucumber

(1) This test was practised in the same manner as the test on Botrytis rot (gray mold) of cucumber, except for using the mycelium of *Sclerotinia schlerotiorum* as inoculum.

(2) Evaluation
The same as in test 2.

(3) Results

Table 3

| Test Compound | Concentration (ppm) | Disease Control (%) | |
|---|---|---|---|
| | | Protective Spraying | Curative Spraying |
| 3 | 125 | 100 | 95 |
| 5 | 125<br>31.3 | 100<br>100 | 97<br>96 |
| 7 | 125<br>31.3 | 100<br>89 | 96<br>94 |
| Iprodione | 125<br>31.3 | 100<br>100 | 90<br>77 |
| Untreated | - | 0 | 0 |

Test 4 Test of powdery mildew of cucumber.

(1) Seeds of cucumber (Cultivar: Matsukaze) were sown in pots of 9 cm in diameter and the pots were kept in a greenhouse until the plants had reached the 2—3 leaf stage. The (i), the first leaf of each plant was sprayed with 3 ml of a solution of the test compound at a prescribed concentration and dried in the air (foliar treatment). Inoculation of a spore suspension of *Sphaerotheca fuliginea* by spraying was practised 2 days before the treatment (curative effect) or 1 day after the treatment (protective effect). Alternatively, (ii) 5 ml of the solution was applied at the root of the seedlings (soil treatment). The pots were kept then in a greenhouse for 2 days and inoculation was practised in the same manner as indicated before. In a third series (iii), each plant was sprayed with 3 ml of a solution of the test compound at a concentration of 125 ppm and dried in the air. The treated pots were set in a greenhouse and untreated pots were placed next to the treated pots. Treated and untreated plants were inoculated then with a spore suspension in order to check the protective effect of the test compounds by means of their vapors. The effect was examined 14 days after the inoculation with spores.

(2) Evaluation

The results of both foliar and soil treatments were represented by the following indexes:

0: signs of disease observed on the whole leaf
3: signs of disease observed on about 50% of the leaf
5: signs of disease observed on about 30% of the leaf
7: signs of disease observed on about 20% of the leaf
9: a small sign of disease observed
10: no sign of disease observed.

The presence or absence of a vapor effect is shown by position (+) or negative (−) indications which correspond to indexes of 9—10 and 0—3 in the above list, respectively.

(3) Results

Table 4

| Test Compound | Concentration (ppm) | Protective Index | | Vapor effect |
|---|---|---|---|---|
| | | Foliar Treatment | Soil Treatment | |
| 1 | 125 | 10 | 3 | + |
| 3 | 125 | 10 | 10 | + |
| Chinome-thionat | 125 | 10 | - | - |
| Dimethiri-mol | 500 | - | 5 | - |
| Untreated | - | 0 | 0 | - |

Table 5

| Test Compound | Concentration (ppm) | Foliar Treatment | | Soil Treatment |
|---|---|---|---|---|
| | | Protective Effect | Curative Effect | Protective Effect |
| 4 | 31.3<br>7.8<br>2.0<br>0.5 | 10<br>10<br>5<br>0 | 10<br>10<br>3<br>0 | |
| 5 | 31.3<br>7.8<br>2.0<br>0.5 | 10<br>10<br>10<br>5 | 10<br>10<br>10<br>10 | 10<br>10<br>0<br>0 |
| 6 | 31.3<br>7.8<br>2.0<br>0.5 | 10<br>9<br>3<br>0 | 10<br>10<br>5<br>0 | |
| 7 | 31.3<br>7.8<br>2.0<br>0.5 | 10<br>10<br>9<br>0 | 10<br>10<br>10<br>7 | 10<br>10<br>0<br>0 |
| 8 | 31.3<br>7.8<br>2.0<br>0.5 | 10<br>9<br>0<br>0 | 10<br>10<br>10<br>5 | 0<br>0<br>0<br>0 |
| Triadimefon | 31.3<br>7.8<br>2.0<br>0.5 | 10<br>9<br>7<br>0 | 10<br>10<br>9<br>0 | 10<br>3<br>0<br>0 |
| Untreated | - | 0 | 0 | 0 |

Test 5 Test on powdery mildew of cucumber (vapor effect)

(1) The first leaves of young cucumber plants (Cultivar: Matsukaze) planted in pots of 90 cm in diameter were inoculated with a spore suspension of *Spaerotheca fuliginea.* The plants were kept in a greenhouse for 2 days, whereupon thin cover plates, 15 mm in diameter, were laid on the first leaves. Filter paper disks soaked with 80 µl of test solution at a concentration of 31.3, 7.8, 2.0 or 0.5 ppm were laid on the plates for 3 days. The disease degree was observed 14 days after the inoculation.

(2) Evaluation.

The result was evaluated as + when an inhibition zone was observed around the plate, as ± when the inihibition zone had nearly the same diameter (8 mm) as the plate, or as − when there was no inhibition zone.

(3) Results

Table 6

| Test Compound | Concentration (ppm) | Protective Effect by Vapor |
|---|---|---|
| 7 | 31.3<br>7.8<br>2.0<br>0.5 | +<br>+<br>-<br>- |
| Triadimefon | 31.3<br>7.8<br>2.0<br>0.5<br>0.5 | -<br>-<br>-<br>-<br>- |
| Untreated | - | - |

Test 6 Anti-fungal activity *in vitro*

(1) Each test compound was diluted two-fold with GY medium (2% glucose and 0.4% yeast extract) containing 0.01% Tween 80 (trademark). Each diluted solution was inoculated with a spore suspension ($10^{5-6}$ spores/ml) of *Alternaria kikuchiana* or with a hyphae-containing agar disk (4 mm in diameter) of other fungi and incubated at 28°C for 2 days.

(2) Evaluation.

The growth of hyphae was observed with the naked eye and the minimum inhibition concentrations were determined.

TABLE 7

| | MIC (μg/ml) | |
|---|---|---|
| | Test | Compound |
| Test Fungus | 5 | Fenarimol |
| *Monilinia fructicola* | 0.2 | 3.13 |
| *Cochliobolus sativus* | 6.25 | 1.56 |
| *Pyrenophora graminea* | 12.5 | 12.5 |
| *Diaporthe citri* | 25.0 | >50.0 |
| *Ustilago nuda* | 1.56 | 25.0 |
| *Corticium rolfsii* | 50.0 | 25.0 |
| *Alternaria kikuchiana* | 12.5 | 6.25 |
| *Vasla ceratosperma* | ND | 12.5 |
| *Cladosporium herbarum* | 6.25 | >50.0 |

N.D.: Not done

As shown above, the compounds (I) of this invention and their salts have an extremely strong anti-fungal activity and are useful in agricultural and industrial fungicides.

Compounds of formula I can be prepared by reacting an azolyl cycloheptanone derivative of formula II with a Grignard reagent or an alkali metal compound of formula III in order to introduce a substituent R in 1-position of the cycloheptane ring. This reaction can be represented by the following scheme:

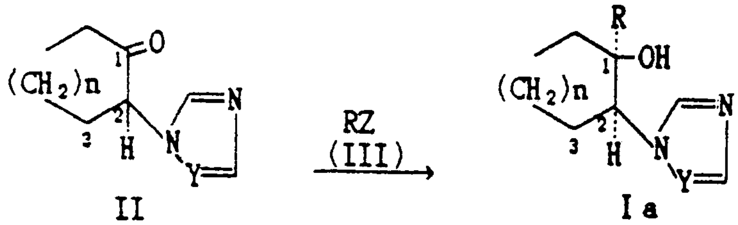


II

Ia

wherein R, Y and n have the same meanings as in formula I, and Z is a halomagnesium group or an alkali metal atom. The main product is a 1-substituted azolylcycloheptanol derivative having its azolyl and hydroxyl groups in cis-configuration (formula Ia).

The reaction can be effected in an ether solvent (e.g. tetrahydrofuran, diglyme, ether or isopropylether) or in a mixed reaction medium such as a benzene solvent (e.g. benzene or toluene) containing an ether solvent. Peferably, the ether solvent alone is used as a reaction medium. The reaction is generally effected under cooling.

Among the starting compounds of formula II, (1,2,4-triazol-1-yl)cycloheptanone is generally disclosed in GB—A—1.464.224. Other compounds of formula II can easily be prepared as shown by the following reaction scheme:

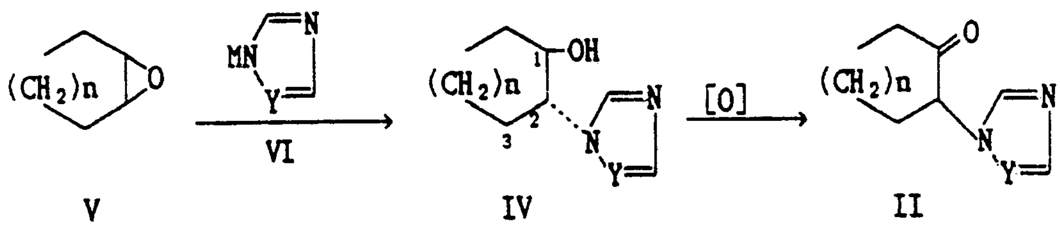


V

IV

II

wherein M is hydrogen or an alkali metal and Y and n each have the same meaning as in formula I.

The reaction of cycloheptene oxide of formula V with imidazole, 1,2,4-triazole or its alkali metal salt (e.g. sodium or potassium salt) of formula VI is effected in a solvent or without solvent at room temperature or under heating to give a 2-azolylcycloheptanol of formula IV. Preferred reaction solvents are polar organic solvents such as alcohols (e.g. methanol, ethanol, propanol, isopropanol), dimethylformamide, or dimethylacetamide. Dimethylformamide and dimethylacetamide are favourable when the compound of formula VI is an alkali metal salt. The reaction may be effected in the presence of a Lewis acid when the compound of formula VI is a free base.

The resulting compound of formula IV is oxidized to give the desired 2-azolylcycloheptanone (II). The oxidation can be effected for example by reaction with an acid anhydride (e.g. trifluoroacetic anhydride or acetic anhydride) or oxalyl chloride and dimethylsulfoxide in methylene chloride, followed by treatment with an organic base (e.g. triethylamine or pyridine).

The resulting compound of formula II can be separated and purified by conventional methods such as extraction, recrystallization and chromatography and may be converted to a desired salt, if necessary.

The desired compound of formula I can be prepared by the above process. A halogenated compound of formula I can also be prepared by halogenation of a corresponding non-halogenated compound of the same formula. The resulting compound can be separated and purified by conventional methods such as extraction, recrystallization, and chromatography and can be converted to a desired salt, if necessary.

The compounds (I) can be formulated to an anti-fungal composition for agricultural use by mixing them with a suitable solid or liquid carrier and other suitable adjuvants such as surfactants, diluents, spreaders, synergists, stickers and dispersants. Solid carriers include talc, clay, bentonite, pyrophyllite, kaolin, diatomaceous earth and silica. Liquid carriers include water, methanol, ethanol, acetone, dimethylformamide, ether, benzene, xylene, toluene and naphtha. Surfactants include non-ionic surfactants (e.g. polyoxyethylene alkyl phenyl ethers and polyoxyethylene fatty acid esters) and anionic surfactants (e.g. alkylbenzene sulfonic acid salts, lignin sulfonic acid salts and dinaphthylmethane sulfonic acid salts). As stickers, polyvinyl alcohols, CMD and gum arabic may be used. The concentration of the active ingredient may be from 0.01 to 90 weight% based on the weight of the composition.

The anti-fungal composition can have the shape of powders, wettable powders, granules, emulsifiable concentrates, suspensions, solutions, fumigants, gases or pastes and can be used for sterilizing agricultural products (including seedlings and seeds) as well as for sterilizing the soil. Thus, for example, a compound

(I) is homogeneously dissolved into a hydrocarbon or an alcohol with a suitable surfactant to give an emulsifiable concentrate or a solution. Or, the compound (I) is mixed with a mineral powder and with a suitable surfactant, crushed and homogenized to give a wettable powder. The thus-prepared composition can be diluted with water to a desired concentration and applied. Alternatively, the compound (I) may be diluted with mineral powder, homogeneously crushed, blended and used as a dust. Each composition may be diluted to contain an effective amount of compound I. Further, the compositions can be combined with other agrochemicals, e.g. insecticides, sterilizers, herbicides, plant-growth regulators and miticides. It also can be mixed with nutrients.

The composition can be used at lower concentrations than the fungicides on the market as shown in the above tests. The concentration is, for example, about 10 to about 1.000 ppm, preferably about 5.0 to about 500 ppm when used for protective or curative spraying.

The compounds (I) can sometimes be applied by soaking a suitable material such as paper, rope or cloth with one of the above compositions and then arranging this material around plants. The compounds will work then by means of their vapor effect.

Additionally, compositions containing compounds (I) may be used as industrial sterilizers, for example, for painting, timber, paper and cloth. Thus, an effective amount of compound (I) is mixed with a ship's paint to prevent adherence of shellfish and algae. Or, a sterilizer containing a compound (I) at an effective concentration may be sprayed onto wall-paper or wall-cloth of this material may be soaked with it.

The following examples are included merely to aid in the understanding of the invention. The term THF means tetrahydrofuran.

Example 1

(1) Cycloheptene (10 g) was dissolved in methylene chloride (400 ml) and 85% m-chloroperbenzoic acid (23 g) was added thereto in portions with stirring under ice-cooling. The mixture was stirred for 10 hours under ice-cooling and at room temperature for 3 hours. The precipitated benzoic acid was filtered off and washed with methylene chloride. The filtrate and the washings were combined and washed with an aqueous solution of sodium thiosulfate, 5% potassium carbonate and water successively. After drying, an evaporation of methylene chloride gave cycloheptene oxide (V) as an oil.

To the product were added propanol (25 ml) and 1,2,4-triazole (8,6 g). The mixture was heated for 4 hours at 100°C and the propanol was removed by evaporation. After addition of a small amount of water, the residue was extracted with chloroform. The extract was washed with a small amount of water, dried, and evaporated. The resultant residue was recrystallized from ether-hexane to give 2-(1,2,4-triazol-1-yl)cycloheptanol (IVa) (13.3 g) (Yield: 35.8%), Mp. 85—86°C.

NMR: δ ($CDCl_3$) 1.6—2 ml OH, 4 (br) 2H, 5.0 s 1H, 7.73 s 1H, 8.03 s 1H.

(2) A solution of dimethylsulfoxide (1.05 g) in methylene chloride (10 ml) was cooled to −78°C and a solution of trifluoroacetic anhydride (1.65 ml) in methylene chloride (4 ml) was added dropwise thereto. The mixture was stirred for 1 hour under cooling. A solution of Compound IVa (1.63 g) in methylene chloride (18 ml) was added dropwise thereto and the mixture was made to react at −78°C to room temperature for 1.5 hours and then cooled to −65°C. Triethylamine (3.75 ml) was added thereto and the mixture was stirred at −65°—0°C for 1 hour. The reaction mixture was made alkaline with 5% potassium hydroxide and extracted with methylene chloride. The extract was washed with water, dried and evaporated. The residue was applied to column chromatography on silica gel (30 g). From a chloroform-methanol (100:1) fraction, 2-(1,2,4-triazol-1-yl)-cycloheptanone (IIa) (1.16 g) was obtained (yield 72%). Mp. 82—82.5°C (ether-hexane).

IR: $\nu_{max}$ ($CHCl_3$) 1720 $cm^{-1}$

NMR: δ ($CDCl_2$) 1.5—2.3 m 8H, 2.65 m 2H, 5.28 d-d (J=12,5) 1H, 7.91 s 1H, 8.22 s 1H

(3) To a Grignard reagent prepared from anhydrous ether (10 ml), magnesium (150 mg) and p-chlorobenzyl chloride (966 mg) was added a solution of Compound IIa (538 mg) in THF (10 ml) with stirring under ice-cooling. The mixture was made to react at room temperature for 1 hour and after addition of water, was extracted with chloroform. The extract was washed with water, dried and evaporated. The resultant residue was applied to column chromatography on silica gel (15 g). From an ether fraction, cis-1-(4-chlorobenzyl)-2-(1,2,4-triazol-1-yl)cycloheptanol (*1*) was obtained as crystals (yield 75%). MP. 111—112°C (ether-hexane).

NMR: δ ($CDCl_3$) 1.63 m 10H, 2.42 q (J=14) 2H, 3.63 s 1H, 4.23 d-d (J=12, −1) 1H 7.01 d (J=9) 2H, 7.22 (J=9) 2H, 7.95 s 1H, 8.20 s 1H.

| | | | | |
|---|---|---|---|---|
| Anal. Calcd. for $C_{15}H_{20}NOCl$: | C, 62.84; | H, 6.59; | N, 13.74; | Cl, 11.59 (%) |
| Found: | C, 62.74; | H, 6.46; | N, 13.54; | Cl, 11.61 (%) |

Example 2

The following compound of formula Ia was obtained in the same manner as in Example 1

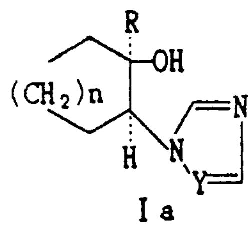

Ia

Table 8

| Example No. | R | n | Y | Mp(°C) | NMR: δ($CDCl_3$) |
|---|---|---|---|---|---|
| 2 | 2-thienyl | 3 | N | 135-6 | 4.45d-d(J=11,-1) 5.1s |

Example 3

To a solution of cis-1-(2-thienyl)-2-(1,2,4-triazol-1-yl)cycloheptanol (*2*) (790 mg)] as obtained in Example 2 in methylene chloride (25 ml) was added 1.8M chlorine-carbon tetrachloride solution (2.5 ml) under ice-cooling. The mixture was allowed to react for 10 minutes. After addition of a sodium hydrocarbonate solution, the mixture was extracted with methylene chloride. The extract was washed with water and evaporated. The residue was applied to column chromatography on silica gel (15 g). From the ether fraction, cis-1-(5-chloro-2-thienyl)-2-(1,2,4-triazol-1-yl)cycloheptanol (*3*) (585 mg) was obtained. Mp. 170—173°C.

NMR: ($CDCl_3$) 4.38 d (J=11, −1) 1H, 5.13 s 1H.

Example 4

(1) To a solution of cycloheptene (VII) (10 g) in methylene chloride (400 ml) was added 85% m-chloroperbenzoic acid (23 g) in small portions with stirring under ice-cooling. The mixture was allowed to react at 3—10°C for 10 hours and at room temperature for 3 hours and after removal of the precipitated benzoic acid by filtration, it was washed with methylene chloride. The filtrate and washings were combined and washed with an aqueous solution of sodium thiosulfate, 5% potassium carbonate and water successively. Then, they were dried and evaporated to give cycloheptene oxide (V).

A mixture of the product with propanol (30 ml) and imidazole (8.5 g) was refluxed for 20 hours under heating whereupon the propanol was removed by evaporation. After addition of a small amount of water, the reaction mixture was extracted with chloroform. The extract was washed with a saturated aqueous solution of sodium chloride, dried and evaporated to give an oily residue. The residue was applied to column chromatography on silica gel (60 g). From a chloroform fraction, the starting material (4.41 g) was recovered and from a chloroform-methanol (50:1) fraction, trans-2-imidazolylcycloheptanol (IVb) (3.96 g) was obtained as an oil (yield 21%).

NMR: δ ($CDCl_3$) 1.75 m 10H, 2.12 br 2H, 5.27 s 1H, 6.77 s 1H, 6.83 s 1H, 7.27 s 1H ppm.

(2) Dimethylsulfoxide (2.75 g) was mixed with methylene chloride (20 ml), and cooled to −76°C with stirring, whereupon a diluted solution of anhydrous trifluoroacetic acid (6.0 g) in methylene chloride (10 ml) was added dropwise thereto. The mixture was stirred for 1.5 hours under cooling and a solution of Compound IVb (3.96 g) in methylene chloride (25 ml) was added dropwise thereto. The mixture was allowed to react at −76—0°C for 1 hour, cooled to −60°C and after dropwise addition of triethylamine (9 ml), stirred for 1 hour at −60—5°C. Water was added to the reaction mixture. The mixture was made alkaline with 5% sodium hydroxide and extracted with methylene chloride. The extract was washed with water, dried and evaporated. The residue was applied to column chromatography on silica gel (40 g). From a chloroform-methanol fraction (100:1), 2-imidazolylcycloheptanone (IIb) (0.77 g) was obtained. Mp. 68—69°C.

IR: $\nu_{max}$ ($CHCl_3$) 1720 $cm^{-1}$

NMR: δ ($CDCl_3$) 1.4—2.2 m 8H, 2.57 m 2H, 4.97 d-d (J=8, 5) 1H, 6.85 d (J=1) 1 H, 7.38 d (J=1) 1H ppm.

(3) To a solution of a Grignard reagent prepared from anhydrous THF (6 ml), and magnesium (45 mg) and p-fluorobromobenzene (200 mg) was added dropwise a solution of Compound IIb (200 mg) in THF (8 ml) under refluxing. The mixture was refluxed for 1 hour with stirring and then evaporated. After addition of water, the residue was extracted with chloroform. The extract was washed with water, dried and evaporated. The residue was applied to column chromatography on silica gel (6 g). From a chloroform-methanol (50:1) fraction, cis-1-(4-fluorophenyl)-2-imidazolylcycloheptanol (*1*) (180 mg) was obtained as crystals. Mp. 164—165°C.

NMR: δ ($CDCl_3$) 1.7—2.6 m 10H, 3.98 d-d (J=11, 2) 1H, 3.9 br 1H, 6.36 brs 2H, 6.7—7.2 m 5H ppm.

Example 5

(1) Cycloheptene oxide (V) prepared from cycloheptene (10 g) in the same manner as in Example 1(1) was allowed to react with propanol (25 ml) and 1,2,4-triazole (8.6 g) at 100°C for 4 hours whereupon the propanol was removed by evaporation. After addition of a small amount of water, the reaction mixture was extracted with chloroform. The residue was washed with a saturated aqueous solution of sodium chloride, dried and evaporated. The residue was recrystallized from ether-hexane to give 2-(1,2,4-triazole-1-yl)cycloheptanol (IVa) (13.3 g) melting at 85—86°C (yield 35.8%).

NMR: δ ($CDCl_3$), 1.6—2 m 10H, 4 br 2H, 5.0 s 1H, 7.73 s 1H, 8.03 s 1H ppm.

(2) A solution of dimethylsulfoxide (1.05 g) of methylene chloride (10 ml) was cooled to −78°C and a solution of trifluoroacetic anhydride (1.65 ml) in methylene chloride (4 ml) was added dropwise thereto with stirring. The mixture was stirred for 1 hour under cooling. A solution of Compound IVa (1.63 g) in methylene chloride (18 ml) was added dropwise thereto. The mixture was allowed to react at −76—100°C for 1.5 hours, then cooled to −65°C, mixed with triethylamine (3.75 ml) and stirred at −65—0°C for 1 hour. The reaction mixture was made alkaline with 5% potassium hydroxide and extracted with methylene chloride. The residue was washed with water, dried and evaporated. The residue was applied to column chromatography on silica gel (30 g). From a chloroform-methanol (100:1) fraction, 2-(1,2,4-triazol-1-yl)cycloheptanone (IIa) 1.16 g) was obtained (yield 72%). Mp. 82—82.5°C (ether-hexane).

IR: $\nu_{max}$ ($CHCl_3$) 1720 $cm^{-1}$

NMR: δ ($CDCl_3$) 1.5—2.3 m 8H, 2.65 m 2H, 5.28 d-d (J=12, 5) 1H, 7.91 s 1H, 8.22 s 1H ppm.

(3) To a Grignard reagent prepared from anhydrous THF (20 ml) and magnesium (835 mg) and p-chlorobromobenzene (6.57 g), was added dropwise a solution of Compound IIa (4.1 g) in THF (35 ml) under cooling. The mixture was made to react at room temperature for 1 hour. After addition of water, it was acidified with 5% hydrochloric acid, made alkaline with 5% potassium carbonate solution and then extracted with benzene. The extract was washed with water, dried and evaporated. The residue was applied to column chromatography on silica gel (70 g).

From an ether fraction, cis-1-(4-chlorophenyl)-2-(1,2,4-triazol-1-yl)cycloheptanol (*5*) (3.6 g) was obtained as crystals (yield 54%). Mp. 129—131°C (acetone-hexane).

| | | | | |
|---|---|---|---|---|
| Anal. calcd. for $C_{15}N_{18}N_3OCl$: | C, 61.75; | H, 6.22; | N, 14.40; | Cl, 12.15 (%) |
| Found: | C, 61.71; | H, 6.22; | N, 14.31; | Cl, 12.45 (%) |

Examples 6—8

The following compounds were obtained by the same procedure as in the above examples.

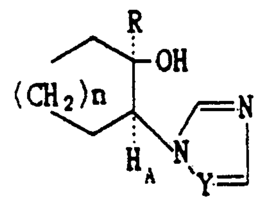

Ia

Table 9

| Example No. | R | n | Y | Mp(°C) | NMR: δ(CDCl3) J=Hz $H_A$ | OH |
|---|---|---|---|---|---|---|
| 6 | 4-chloro-phenyl | 3 | CH | 163-4 | 4.0d-d(J=11, 2) | 3.6(Broad) |
| 7 | 4-fluoro-phenyl | 3 | N | 152-3 | 3.98d-d(J=11, -1) | 3.9(broad) |
| 8 | 3,4-di-chloro-phenyl | 3 | N | 178-9 | 4.50d-d(J=11, -1) | 4.96s |

Example 9

One part of compound *1* is mixed with 99 parts of talc to give a dust.

Example 10

Fifty parts of compound *3*, 6 parts of sodium alkyl benzenesulfonate, 4 parts of sodium ligninesulphonate and 40 parts of clay are mixed and crushed to give a wettable powder. This powder is diluted so that the effective concentration of compound *3* is 10—500 ppm and sprayed to fruit.

Example 11

Fifty parts of compound *5*, 6 parts of sodium alkyl benzenesulfonate, 4 parts of sodium lignine sulfonate and 40 parts of clay are mixed and crushed to give a wettable powder. The powder is diluted so that the effective concentration of compound *5* is 50—500 ppm, and sprayed to fruit.

Example 12

Twenty-five parts of compound *7*, 8 parts of polyoxyethylene alkyl phenyl ether, 2 parts of sodium alkyl benzene sulfonate and 65 parts of xylene are mixed and dissolved to give a concentrated emulsion. This emulsion is diluted so that the effective concentration of compound *7* is 50—5000 ppm and sprayed on leaves.

Example 13

A wettable powder is prepared in the same manner as in Example 11 using compound *8* instead of compound *5*.

**Claims**

1. Compounds of the formula:

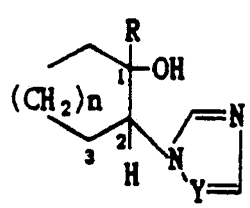

I

wherein R is a phenyl, benzyl or thienyl group optionally substituted by one or more halogen atoms, Y is a methine or nitrilo group and n is 3, with the proviso that the azolkyl and hydroxyl groups attached to the cycloheptane ring are in *cis*-configuration; as well as their salts.

2. A compound as claimed in claim 1 wherein R is a benzyl or thienyl group optionally substituted by one or more halogen atoms.

3. A compound as claimed in claim 1 wherein R is a phenyl group optionally substituted by one or more halogen atoms.

4. A compound as claimed in claim 1 wherein the halogen is chlorine or fluorine.

5. A compound as claimed in claim 1 wherein R is a 2-chlorophenyl, 2-fluorophenyl, 3-chlorophenyl, 4-chlorophenyl, 4-fluorophenyl, 2,4-dichlorophenyl, 2,5-dichlorophenyl, 3,4-dichlorophenyl, benzyl, 2-chlorobenzyl, 4-chlorobenzyl, 2,4-dichlorobenzyl, 4-fluorobenzyl, 2,4-difluorobenzyl, 2-thienyl, 5-chloro-2-thienyl, 5-iodo-2-thienyl, 3,5-dichloro-2-thienyl, or 3,4,5-trichloro-2-thienyl group.

6. A compound as claimed in claim 5 wherein Y is a nitrilo group.

7. A compound as claimed in claim 1 wherein R is 2-chlorophenyl, 4-chlorophenyl, 2,5-dichlorophenyl, 5-chloro-2-thienyl or 3,4,5-trichloro-2-thienyl group and Y is a nitrilo group.

8. A compound as claimed in claim 1 which is *cis*-1-(4-chlorophenyl)-2-(1,2,4-triazol-1-yl)cycloheptanol.

9. A compound as claimed in claim 1 which is *cis*-1-(5-chloro-2-thienyl)-2-(1,2,4-triazol-1-yl)cycloheptanol.

10. Use of the compound as claimed in claim 1 for preparing a composition useful as an agricultural fungicide.

11. A method of preparing a compound as claimed in claim 1, comprising the step of reacting a compound of the formula:

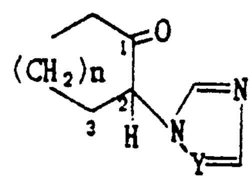

II

with a compound of the formula RZ, wherein R, Y and n have the meanings stated in claim 1 and Z is a halogeno magnesium group or an alkali metal atom.

**Patentansprüche**

1. Verbindungen der Formel:

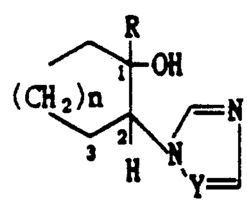

I

dadurch gekennzeichnet, daß R eine Phenyl-, Benzyl- oder Thienylgruppe, gegebenenfalls substituiert durch ein oder mehrere Halogenatome, bedeutet, Y eine Methin- oder Nitrilogruppe und n 3 bedeutet, mit der Maßgabe, daß die Azolyl- und Hydroxylgruppen an dem Cycloheptanring *cis*-konfiguriert sind; ebenso wie ihre Salze.

2. Verbindung nach Anspruch 1, dadurch gekennzeichnet, daß R eine Benzyl- oder Thienylgruppe, gegebenenfalls substituiert durch ein oder mehrere Halogenatome, bedeutet.

3. Verbindung nach Anspruch 1, dadurch gekennzeichnet, daß R eine Phenylgruppe, gegebenenfalls substituiert durch ein oder mehrere Halogenatome, bedeutet.

4. Verbindung nach Anspruch 1, dadurch gekennzeichnet, daß das Halogen, Chlor oder Fluor ist.

5. Verbindung nach Anspruch 1, dadurch gekennzeichnet, daß R eine 2-Chlorphenyl-, 2-Fluorphenyl- 3-Chlorphenyl-, 4-Chlorphenyl-, 4-Fluorphenyl-, 2,4-Dichlorphenyl-, 2,5-Dichlorphenyl-, 3,4-Dichlorphenyl-, Benzyl-, 2-Chlorbenzyl-, 4-Chlorbenzyl, 2,4-Dichlorbenzyl, 4-Fluorbenzyl, 2,4-Difluorbenzyl-, 2-Thienyl-, 5-Chlor-2-Thienyl, 5-Iod-2-Thienyl-, 3,5-Dichlor-2-Thienyl- oder eine 3,4,5-Trichlor-2-Thienylgruppe bedeutet.

6. Verbindung nach Anspruch 5, dadurch gekennzeichnet, daß Y eine Nitrilogruppe bedeutet.

7. Verbindung nach Anspruch 1, dadurch gekennzeichnet, daß R eine 2-Chlorphenyl-, 4-Chlorphenyl-, 2,5-Dichlorphenyl-, 5-Chlor-2-Thienyl- oder 3,4,5-Trichlor-2-Thienylgruppe bedeutet und Y eine Nitrilogruppe bedeutet.

8. Verbindung nach Anspruch 1, dadurch gekennzeichnet, daß sie *cis*-1-(4-Chlorphenyl)-2-(1,2,4-Triazol-1-yl)Cycloheptanol ist.

9. Verbindung nach Anspruch 1, dadurch gekennzeichnet, daß sie *cis*-1-(5-Chlor-2-Thienyl)-2-(1,2,4-Triazol-1-yl)Cycloheptanol ist.

10. Verwendung der Verbindungen nach Anspruch 1 zur Herstellung eines Präparats, das als landwirtschaftliches Fungizid geeignet ist.

11. Verfahren zur Herstellung einer Verbindung nach Anspruch 1, dadurch gekennzeichnet, daß eine Verbindung der Formel

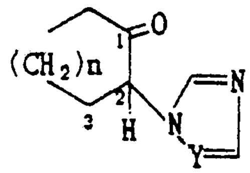

II

mit einer Verbindung der Formel RZ, worin R, Y und n wie in Anspruch 1 definiert sind und Z eine Halogenmagnesium-gruppe oder ein Alkalimetallatom bedeutet, umgesetzt wird.

**Revendications**

1. Composés de formule:

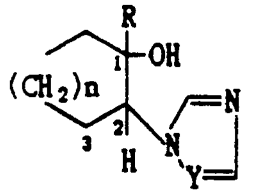

I

où:

R représente un groupe phényle, benzyl ou thiènyle, facultativement substitué par un ou plusieurs atomes d'halogène;

Y représente un groupe méthine ou nitrilo; et

n vaut 3,

à la condition que les groupes azolyle et hydroxyle fixés au noyau cycloheptane se situent en configuration *cis*; ainsi que leurs sels.

2. Composé selon la revendication 1, dans lequel R représente un groupe benzyle ou thiényle, facultativement substitué par un ou plusieurs atomes d'halogène.

3. Composé selon la revendication 1, dans lequel R représente un groupe phényle, facultativement substitué par un ou plusieurs atomes d'halogène.

4. Composé selon la revendication 1, dans lequel l'halogène est le chlore ou le fluor.

5. Composé selon la revendication 1, dans lequel R représente un groupe chloro-2 phényle, fluoro-2 phényle, chloro-3 phényle, chloro-4 phényle, fluoro-4 phényle, dichloro-2,4 phényle, dichloro-2,5 phényle, dichloro-3,4 phényle, benzyle, chloro-2 benzyle, chloro-4 benzyle, dichloro-2,4 benzyle, fluoro-4 benzyle, difluoro-2,4 benzyle, thiényle-2, chloro-5 thiényle-2, iodo-5 thiényle-2, dichloro-3,5 thiényle-2 ou trichloro-3,4,5 thiényle-2.

6. Composé selon la revendication 5, dans lequel Y représente un groupe nitrilo.

7. Composé selon la revendication 1, dans lequel R représente un groupe chloro-2 phényle, chloro-4 phényle, dichlor-2,5 phényle, chloro-5 thiényle-2 ou trichloro-3,4,5-thiényle-2 et Y représente un groupe nitrilo.

8. Composé selon la revendication 1, qui est le *cis*-(chloro-4 phényl)-1 triazol-1,2,4 yl-1)-2-cycloheptanol.

9. Composé selon la revendication 1, qui est le *cis*-(chloro-5 thiényl-2)-1 (triazol-1,2,4 yl-1)-2 cycloheptanol.

10. Utilisation des composés selon la revendication 1 pour la préparation d'une composition utile en tant que fongicide pour l'agriculture.

11. Procédé de préparation d'un composé tel que défini à la revendication 1, comprenant l'étape consistant à faire réagir un composé de formule:

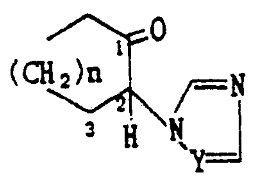

II

avec un composé de formule RZ, où:

R, Y et n ont les significations énconcées à la revendication 1; et

Z représente un groupe halogéno-magnésium ou un atome de métal alcalin.